# EUROPÄISCHE PATENTSCHRIFT

(11) **EP 3 027 166 B1**
(45) Veröffentlichungstag und Bekanntmachung des Hinweises auf die Patenterteilung: **12.02.2020**
(21) Anmeldenummer: 14734474.1
(22) Anmeldetag: 01.07.2014
(51) Int. Cl.: A61K 31/133, A61K 31/198, A61K 8/44, A61P 17/14, A61K 8/68, A61Q 7/00

(54) **FORMULIERUNGEN ENTHALTEND SPHINGANIN**
FORMULATIONS CONTAINING SPHINGANINE
FORMULATIONS CONTENANT DE LA SPHINGANINE

(30) Priorität: 29.07.2013 DE 102013214713
(43) Veröffentlichungstag der Anmeldung: 08.06.2016
(73) Patentinhaber: Evonik Operations GmbH, 45128 Essen (DE)
(72) Erfinder: KÖHLER, Tim, 46284 Dorsten (DE); FARWICK, Mike, 45138 Essen (DE); MENTEL, Matthias, 44357 Dortmund (DE); LERSCH, Peter, 46537 Dinslaken (DE)
(74) Vertreter: Evonik Patent Association
(86) Internationale Anmeldenummer: PCT/EP2014/063917
(87) Internationale Veröffentlichungsnummer: WO 2015/014558

(56) Entgegenhaltungen:
- WO-A1-2013/017361
- CH-A- 399 655
- DE-A1- 10 114 561
- FR-A1- 2 820 037
- US-A1- 2004 171 693

## Beschreibung

### Gebiet der Erfindung

Gegenstand der vorliegenden Erfindung sind Zusammensetzungen enthaltend
A) Sphinganin und
B) mindestens einen Wirkstoff ausgewählt aus der Gruppe Kreatin, Kreatin-Hydrate und Di-Natrium Phospho-Kreatin zur Verwendung zur Stimulation des Haar-Wachstums.

### Stand der Technik

Weite Teile der menschlichen Bevölkerung leiden unter Haarausfall, bzw. einem verlangsamten Wachstum der Haare v.a. bei fortgeschrittenem Alter. Im Extremfall äußert sich dieses durch partielle oder vollständige Glatzköpfigkeit.
Das menschliche Haar unterliegt einem komplexen Wachstumszyklus, der sich in drei charakteristische Phasen aufteilen lässt: Einer mehrjährigen Anagenphase, in der das Haar wächst, einer kurzen mehrwöchigen Katagen-Phase (Übergangsphase), sowie einer Telogenphase (Ruhephase), die mehrere Monate dauert. Ist ein Zyklus durchlaufen, folgt im Regelfall ein neuer Zyklus, um das ausgefallene Haar zu ersetzen. Dieser Prozess dauert bei ungestörtem Haarwachstum lebenslang an, kann aber gestört sein.
Entsprechend des komplexen Haarwachstumszyklus sind die Ursachen für den Haarausfall vielfältig und teilweise geschlechtsspezifisch.
Vielfach verkürzen sich die Wachstumsphasen des Haares mit zunehmendem Alter, so dass es zur Bildung kürzerer und/oder feinerer Haare (Vellus-Haar) kommt. Durch Verschiebung des Verhältnisses aktiv wachsender Haare zu Haaren in der Ruhephase (Anagen:Telogen-Verhältnis) kann es zu einer verringerten Haardichte kommen. Im Extremfall kommt der Haarwachstumszyklus vollständig zum Erliegen, was sich dann in dem Phänomen der Glatzköpfigkeit äußert.
Männer sind im Vergleich zu Frauen häufiger von Haarausfall betroffen. Im Gegensatz zu Frauen leiden sie vielfach unter einer geschlechtsspezifischen Form des Haarausfalls, der sogenannten androgenetischen oder androgenen Alopezie. Diese Form des Haarausfalls ist erblich bedingt und tritt oftmals schon in jungen Jahren auf. Hauptursache für diese Form des Haarausfalls ist das Steroidhormon Dihydrotestosteron (DHT). Es wird im menschlichen Körper durch das Enzym Steroid-5-alpha Reduktase aus dem männlichen Geschlechtshormon Testosteron gebildet und führt bei angeborener DHT-Überempfindlichkeit zur Verkürzung der Anagenphase der Haare. Eine andere Form des Haarausfalls, die sogenannte *Alopecia areata,* bezeichnet einen lokal begrenzten, kreisförmigen Haarausfall. Diese Form des Haarausfalls ist eine entzündliche Autoimmunerkrankung und kann in allen Altersstufen auftreten.
Eine Form des Haarausfalls, die v.a. bei Frauen zu beobachten ist, ist die diffuse Alopezie. Hierbei kommt es zu einem zeitlich begrenzten Verlust der Haare, wobei die Ursachen vielfältig sein können, z.B. Hormonschwankungen, Stress oder Eisenmangel.

In der kosmetischen oder pharmazeutischen Industrie sind eine Reihe von Verbindungen beschrieben worden, um die sichtbaren Auswirkungen der Alopezie zu vermindern. Ein Teil der vorgeschlagenen Substanzen kann dem Verlust von Haaren vorbeugen oder diesen zumindest vermindern. Andere Substanzen können das Wachstum der Haare stimulieren, v.a. indem die Haarfollikel zum Wachstum angeregt werden und dadurch ein neuer Haarwachstumszyklus initiiert wird.
Zu den beschriebenen pharmazeutischen Wirkstoffen, die erfolgreich zur Behandlung der Alopezie eingesetzt werden, gehören u.a. Steroidhormone wie z.B. Finasterid oder Dutasterid, Alfatradiol bzw. 17α-Estradiol, sowie Cortison-haltige Präparate. Obwohl positive Wirkungen für diese Substanzen belegt sind, treten oftmals unerwünschte Nebenwirkungen auf, da es sich um pharmakologisch aktive Substanzen handelt. Desweiteren sind die genannten Substanzen oftmals spezifisch nur bei Männern wirksam, nicht jedoch universell einsetzbar. Auch sind die positiven Effekte zumeist unzureichend, d.h. die Alopezie wird nur partiell verhindert bzw. vermindert. Ausserdem sind die positiven Effekte zumeist nur temporär, d.h. nach fortdauernder Anwendung oder bei Absetzung des Präparats wird der Ausgangszustand wiederhergestellt.
Neben den pharmakologisch wirksamen Substanzen sind auch eine Reihe von kosmetischen Wirkstoffen vorgeschlagen worden, um den Haarausfall zu behandeln/das Haarwachstum zu fördern. Dazu gehören verschiedene Vitamine (v.a. Vitamine der B-Reihe, z.B. Vitamin B7 bzw. Biotin), Aminosäuren (v.a. L-Valin, L-Arginin) und Aminosäure-Derivate (z.B. Kreatin, L-Carnitin), welche u.a. den Zellstoffwechsel anregen. Sowie Koffein, welches v.a. über die Stimulation der Mikrozirkulation die Durchblutung der Kopfhaut und indirekt das Haarwachstum anregt. Desweiteren sind eine Reihe von Pflanzenextrakten zur Stimulation des Haarwachstums beschrieben worden, u.a. Extrakte der Sägepalme.

Neben den oben bereits angesprochenen Substanzen sind eine Vielzahl von Molekülen bzw. komplexen Gemischen im Kontext einer Stimulation des Haarwachstums beschrieben worden. Dazu gehören folgende Pflanzenextrakte bzw. Reinstoffe sowie davon abgeleitete Derivate oder Vorstufen:
Acetyltyrosin, Allantoin, Bioquinon (Ubiquinon, Coenzym Q10), Aminosäuren (v.a. Arginin, Serin, Methionin), *Arctium Majus*-Wurzelextrakt, Biotinoyl Tripeptide-1, Bisabolol, *Boswellia serrata*-Extrakt (Weihrauch), L-Carnitin,L-Carnitin-L-Tartrat, Acetyl-Carnitin,Palmitoyl-Carnitin,*Carthamus tinctorius-Glucoside,* Diethyllutidinate (Diethyl 2,4-pyridinedicarboxylate; Stemoxidine), *Echinacea*-Extrakte, Ectoin, *Emblica officianalis* (*Phyllantus emblica*)-Extrakt, Epigallocatechingallat (EGCG), Estron, Fluridil (Eucapil), *Ginkgo Biloba*-Extrakt, Glabranin, Glycyrrhizinsäure, *Kigelia africana*-Extrakt, Kopexil (Aminexil; 2,3-Dihydro-3-hydroxy-2-imino-4-pyrimidinamin), *Lithothamnion Calcareum*-Extrakt, Mineralien (v.a. Magnesium-, Calcium- und Zinksalze), Minoxidil, *Moringa*-Pflanzenextrakte, Nicotinsäure (Vitamin B3, Niacin), Nicotinsäureamid (Niacinamid), Oxaloacetat, *Panax Ginseng*-Wurzelextrakt, Panthenol, Pantothensäure (Vitamin B5), Pantolacton, *Paullinia Cupana-*Extrakt (Guarana), Pflanzenprotein-Hydrolysate, Progesteron, Sägepalmen-Beerenextrakt (*Serenoa*), Salicylsäure, *Salvia Officinalis-*Extrakt, *Sargassum Filipendula*-Extrakt, *Sophora*-Wurzelextrakt, Taurin (2-Aminoethansulfonsäure), Tocopherol (Vitamin E), *Tropaeolum Majus*-Extrakt, Xanthine (u.a. Theophyllin, Theobromin), *Zingiber Officinale*-Wurzelextrakt, Ergothionein, Lycopin, Marine Glycogen, Gluconat-Salze, Indianische Kresse, synthetisches Thymus-Hydrolysat, Trichogen, Teebaum-Öl, Kletten-Extrakt, Gingko Biloba Blätterextrakt, Algenextrakte, Grüntee-Extrakt.

Allerdings hat bisher noch keine der o.g. Substanzen dauerhaft zufriedenstellende Ergebnisse für die Stimulation des Haarwachstums gezeigt, so dass die Notwendigkeit zur Entwicklung neuartiger kosmetischer Wirkstoffe zur effizienteren Stimulation des Haarwachstums bzw. der Verminderung des Haarausfalls ungebrochen ist. Bevorzugt werden dabei natürliche, körpereigene Verbindungen.

EP0790053 beschreibt die Verwendung eines 2-Aminoalkan-1,3-Diols zur Verlangsamung des Haarausfalls und/oder zum Auslösen und Stimulieren des Haarwachstums. Dabei wurde gezeigt, dass die Behandlung von in Kultur gehaltenen *ex vivo* Haarfollikeln mit Sphinganin die Lebensdauer der Haarfollikel steigert. Ferner wurde eine wachstumsfördernde Wirkung von Sphinganin auf *in vitro* kultivierte Keratinozyten demonstriert.

DE102011109546 beschreibt die Verwendung von Sphinganin zur Verbesserung des visuellen Erscheinungsbildes von Haut und Haar.

CA2678135 offenbart eine Methode zur Stimulation des Wachstums von Haarfollikeln durch Applikation einer Formulierung enthaltend Kreatin. Die Methode kann dazu eingesetzt werden, dem männlichen androgenetischen Haarausfall, altersbedingtem Haarausfall oder Haarausfall ausgelöst durch Chemotherapie oder Exposition mit Medikamenten vorzubeugen.
Weitere Evidenzen für stimulierende Effekte von Kreatin auf das Haarwachstum finden sich beispielhaft in US20040171693, EP2140857 und WO2012084876.

Positive Effekte von Koffein auf das Wachstum von Haaren sind allgemein bekannt. Der Stand der Technik wird u.a. in folgenden Dokumenten beschrieben: Herman A, Herman A P; Skin Pharmacology and Physiology, Vol 26 (1), 8-14, 2013. Fischer, TW, Hipler, UC, Elsner, P; International Journal of Dermatology Vol 46 (1), 27-35, 2007. Weitere Beispiele finden sich in DE202005011009 und WO2012084876.

Förderliche Effekte von L-Carnitin bzw. L-Carnitin-Derivaten werden in folgenden Dokumenten beschrieben: Foitzik, K, Hoting, E, Heinrich, U, Tronnier, H, Paus, R; Journal of Dermatological Science 48 (2), 141-144, 2007. DE202005011009.

Der Einsatz von Biotin, z.B. zur Stimulation des Haarwachstums oder zur Verbesserung der Struktur der Haare, ist weitverbreitet. Eine Übersicht findet sich beispielhaft in folgendem Review: Noser, F, Bimczok, R; SOFW Journal 122 (8), 511-515, 1996.

Auch die topische Anwendung von Minoxidil zur Stimulation des Haarwachstums ist weitverbreitet. Der Stand der Technik wird beispielhaft in folgendem Review beschrieben: Rossi, A, Cantisani, C, Melis, L, lorio, A, Scali, E, Calvieri, S; Recent Patents on Inflammation & Allergy Drug Discovery 6 (2), 130-136, 2012.

Gleichermassen ist der Einsatz von Arginin zur Stimulation des Haarwachstums bekannt. Ein Beispiel hierfür findet sich in DE20315174.

Aufgabe der Erfindung war es, Formulierungen bereitzustellen, welche Haarwachstum anregen und überragende sensorische Eigenschaften aufweisen.

### Beschreibung der Erfindung

Überraschenderweise wurde gefunden, dass die im Folgenden beschriebenen Wirkstoffkombinationen die der Erfindung gestellte Aufgabe zu lösen vermögen.

Gegenstand der vorliegenden Erfindung sind daher Zusammensetzungen, enthaltend
A) Sphinganin und
B) mindestens einen Wirkstoff ausgewählt aus der Gruppe Kreatin, Kreatin-Hydrate und Di-Natrium Phospho-Kreatin zur Verwendung zur Stimulation des Haar-Wachstums, insbesondere auf Kopfhaut, bevorzugt der eines Menschen.

Ein Vorteil der Erfindung ist es, dass die Formulierungen überlegene sensorische Eigenschaften aufweisen, die zu einem verbesserten Hautgefühl und/oder Haargefühl führen.
Ein weiterer Vorteil der vorliegenden Erfindung ist es, dass die Formulierungen *in vitro* humane follikuläre dermale Papillen-Zellen (HFDPCs) zur Proliferation anregen, was *in vivo* einer Stimulation des Haarwachstums gleichkommt. Erstaunlicherweise erwies sich dabei die Kombination von Sphinganin mit jeweils einem der zusätzlichen Wirkstoffe als deutlich effektiver im Vergleich zur Summe der Induktionen der Zellproliferation, die durch die jeweiligen Einzelsubstanzen erzielt werden konnte.
Ein weiterer Vorteil der vorliegenden Erfindung ist es, dass die Formulierungen verglichen zu den Einzelkomponenten eine verbesserte Verteilbarkeit aufweisen.

Ein weiterer Vorteil der vorliegenden Erfindung ist es, dass die Formulierungen verglichen zu den Einzelkomponenten eine verbesserte Absorption aufweisen. Ein weiterer Vorteil der vorliegenden Erfindung ist es, dass die Formulierungen verglichen zu den Einzelkomponenten eine verminderte Öligkeit aufweisen.
Ein weiterer Vorteil der vorliegenden Erfindung ist es, dass die Formulierungen verglichen zu den Einzelkomponenten eine verminderte Wachsigkeit aufweisen. Ein weiterer Vorteil der vorliegenden Erfindung ist es, dass die Formulierungen verglichen zu den Einzelkomponenten eine verbesserte Gleitfähigkeit aufweisen. Ein weiterer Vorteil der vorliegenden Erfindung ist es, dass die Formulierungen verglichen zu den Einzelkomponenten eine verminderte Klebrigkeit aufweisen. Ein weiterer Vorteil der vorliegenden Erfindung ist es, dass die Formulierungen verglichen zu den Einzelkomponenten eine verbesserte Seidigkeit/Samtigkeit aufweisen.

Alle angegebenen Prozent (%) sind, wenn nicht anders angegeben, Massenprozent.

Bevorzugt sind die folgenden Formulierungen von den erfindungsgemäßen ausgenommen:

| | |
|---|---|
| Laureth-4 | 0.5% |
| PEG-40 Hydrogenated Castor Oil | 0.5% |
| Sphinganine | 0.1% |
| Quaternium-80 | 0.4% |
| Dimethicone Propyl PG-Betaine | 0.6% |
| Cetrimonium Chloride | 0.8% |
| Water | ad 100.0% |
| Creatine | 0.5% |
| Ethanol | 15.0% |
| PVP/VA Copolymer | 4.0% |
| Sodium Hydroxide (10% in water) | 1.2% |

| | | | | |
|---|---|---|---|---|
| Decyl Oleate | 5.7% | 5.7% | 5.7% | 5.7% |
| Ethylhexyl Stearate | 7.3% | 7.3% | 7.3% | 7.3% |
| Glyceryl Stearate | 0.5% | 0.5% | 0.5% | 0.5% |
| Stearic Acid | 0.7% | 0.7% | 0.7% | 0.7% |
| Ceteareth-25; Glycerin; Cetyl Alcohol; Behenic Acid; Cholesterol; Ceramide EOP; CeramideEOS; Ceramide NP; Ceramide NS; Ceramide AP; Caproyl-Phytosphingosine; Caproyl-Sphingosine. | - | 0.5% | - | 0.5% |
| Salicyloyl Phytosphingosine | - | - | 0.05% | 0.03% |
| Creatine | 0.5% | 0.2% | 0.1% | 0.2% |
| Cetearyl Glucoside | 1.0% | 1.0% | 1.0% | 1.0% |
| Glycerin | 3.0% | 3.0% | 3.0% | 3.0% |
| Carbomer | 0.2% | 0.2% | 0.2% | 0.2% |
| Sodium Hydroxide (10%) | 0.7% | 0.7% | 0.7% | 0.7% |
| Ethanol | 9.5% | 5.7% | 3.8%% | 7.6% |
| Sphinganine | 0.33% | 0.20% | 0.13% | 0.26% |
| Water | ad 100.0% | ad 100.0% | ad 100.0% | ad 100.0% |

| | | |
|---|---|---|
| Cetyl PEG/PPG-10/1 Dimethicone | 2.0% | 2.0% |
| Microcristalline Wax | 0.5% | 0.5% |
| Hydrogenated Castor Oil | 0.5% | 0.5% |
| Decyl Oleate | 9.0% | 9.0% |
| Carrylic/Capric Triglyceride | 10.0% | 10.0% |
| Diethylhexyl Carbonate | 5.0% | 5.0% |
| PPG-3 Myristyl Ether; Salicyloyl Phytosphingosine | 3.0% | 3.0% |
| Sodium Chloride | 0.5% | 0.5% |
| Creatine | 0.2% | 0.1% |
| Betaine | - | 0.3% |
| Urea | - | 0.1% |
| Ethanol | 1.9% | 2.85% |
| Sphinganine | 0.07% | 0.1% |
| Water | ad 100.0% | ad 100.0% |

| | |
|---|---|
| Disodium PEG-5 Laurylcitrate Sulfosuccinate; Sodium Laureth Sulfate | 8,0% |
| Sodium Cocoamphoacetate | 12,0% |
| Capryl/Capramidopropyl Betaine | 2,0% |
| Polyglyceryl-3 Caprate | 0,3% |
| PPG-3 Myristyl Ether | 0,5% |
| Sphinganine | 0,1% |
| Water | 77,2% |
| D-Panthenol | 0,2% |
| Creatine | 0,5% |
| Methoxy PEG/PPG-7/3 Aminopropyl Dimethicone | 0,2% |

Erfindungsgemäße Zusammensetzung enthalten Komponente A) bevorzugt in einer Konzentration von 0,01 Gew.-% bis 5,0 Gew.-%, bevorzugt von 0,05 Gew.-% bis 1,0 Gew.-%, besonders bevorzugt von 0,1 Gew.-% bis 0,5 Gew.-%, wobei sich die Gewichtsprozente auf die Gesamtzusammensetzung beziehen.
Erfindungsgemäße Zusammensetzungen enthalten Komponente B) bevorzugt in einer Konzentration von 0,01 Gew.-% bis 20 Gew.-%, bevorzugt von 0,1 Gew.-% bis 10,0 Gew.-%, besonders bevorzugt von 1,0 Gew.-% bis 5,0 Gew.-%, wobei sich die Gewichtsprozente auf die Gesamtzusammensetzung beziehen.
Das Gewichtsverhältnis der Komponente A) zu der Komponente B) in der erfindungsgemäßen Zusammensetzung beträgt-bevorzugt 1 zu 1 bis 1 zu 2000, bevorzugt von 1 zu 2 bis 1 zu 200, besonders bevorzugt von 1 zu 10 bis 1 zu 50.

Die erfindungsgemäßen Zusammensetzungen können z.B. mindestens eine weitere, zusätzliche Komponente enthalten, ausgewählt aus der Gruppe der
Emollients,
Co-Emulgatoren,
Verdicker/Viskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
kosmetische Wirkstoffe,
Pflegeadditive,
Überfettungsmittel,
Lösungsmittel.
Substanzen, die als beispielhafte Vertreter der einzelnen Gruppen eingesetzt werden können, sind dem Fachmann bekannt und können beispielsweise der deutschen Anmeldung DE 102008001788.4 entnommen werden. Diese Patentanmeldung wird hiermit als Referenz eingeführt und gilt somit als Teil der Offenbarung.
Bezüglich weiterer fakultativer Komponenten sowie der eingesetzten Mengen dieser Komponenten wird ausdrücklich auf die dem Fachmann bekannten einschlägigen Handbücher, z. B. K. Schrader, "Grundlagen und Rezepturen der Kosmetika", 2. Auflage, Seite 329 bis 341, Hüthig Buch Verlag Heidelberg, verwiesen.
Die Mengen der jeweiligen Zusätze richten sich nach der beabsichtigten Verwendung. Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.
Da die erfindungsgemäßen Formulierungen das Haarwachstum in unerwartetem Maße fördern, ist ein weiterer Gegenstand der Erfindung die nicht-therapeutische kosmetische Verwendung einer Zusammensetzung enthaltend
A) Sphinganin und
B) mindestens einen Wirkstoff ausgewählt aus der Gruppe Kreatin, Kreatin-Hydrate und Di-Natrium Phospho-Kreatin zur Stimulation des Haar-Wachstums.

Ebenso beugen die erfindungsgemäßen Zusammensetzungen Haarausfall generell vor, somit ist ein weiterer Gegenstand der Erfindung eine Zusammensetzung enthaltend
A) Sphinganin und
B) mindestens einen Wirkstoff ausgewählt aus der Gruppe Kreatin, Kreatin-Hydrate und Di-Natrium Phospho-Kreatin zur Verwendung zur Behandlung von Haarausfall.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Folgende Abbildungen sind Bestandteil der Beispiele:
Abbildung 1: Synergistische Induktion der Zellproliferation durch Sphinganin in Kombination mit Kreatin bzw. Koffein. EtOH, Ethanol; Sa, Sphinganin; Krea, Kreatin; Kof, Koffein.
Abbildung 2. Bewertungsskala Sensorik-Panel

### Beispiele:

### Beispiel 1: Stimulation der Proliferation von humanen follikulären dermalen Papillen-Zellen (HFDPCs) durch Sphinganin in Kombination mit Koffein oder Kreatin.

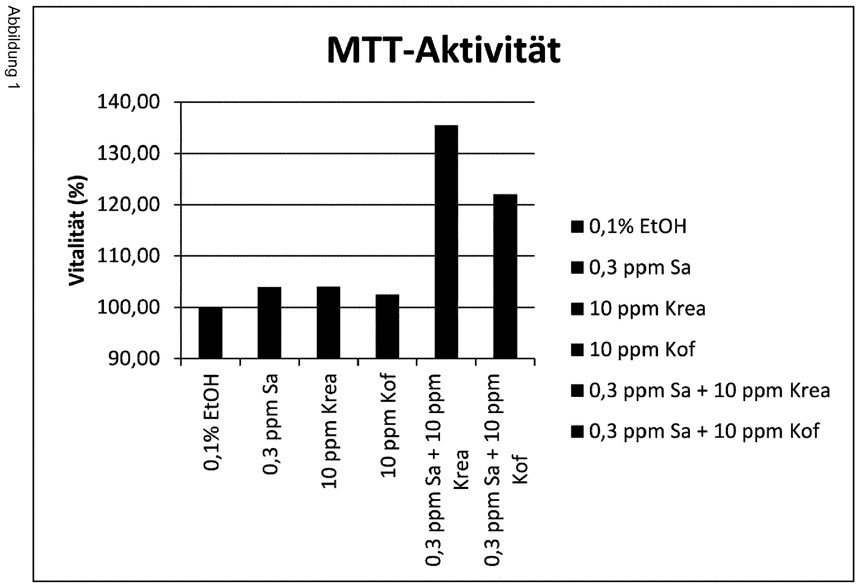

HFDPCs, Medien und sonstige Chemikalien für die Zellkultivierungsversuche wurden von Promocell (Heidelberg, D) bezogen. Die Zellen wurden in einer Zelldichte von ca 6300 Zellen/cm² in einer T75-Flasche mit Follicle Dermal Papilla Cell Growth Medium (kurz: Wachstumsmedium) ausgesät und anschliessend bei 37 ° C, 5% CO₂ inkubiert. Nach 1 Tag erfolgte ein Medienwechsel. Nach weiteren 3 Tagen wurde das Medium entfernt, die Zellen mit PBS (Phosphate Buffered Saline) (ohne Mg²⁺ und Ca²⁺) gewaschen, durch Accutase-Behandlung abgelöst, die Zellzahl bestimmt und anschliessend die Zellen in einer Konzentration von ca 5500 Zellen/cm² in neuen T75-Flaschen in Wachstumsmedium ausgesät und unter gleichen Bedingungen wie oben beschrieben weiterkultiviert. Nach 1 und 3 Tagen erfolgte ein Medienwechsel. Nach 1 weiteren Tag wurde das Medium entfernt, die Zellen in PBS (ohne Mg²⁺ und Ca²⁺) gewaschen, durch Accutase-Behandlung abgelöst und anschliessend in einer Konzentration von ca 10000 Zellen/cm² in die Vertiefungen von 6-well-Zellkulturplatten mit jeweils 2,5 ml Wachstumsmedium ausgesät. Nach 1 Tag erfolgte ein Medienwechsel, und die Prüfsubstanzen - verdünnt in frischem Medium - wurden appliziert. Folgende Konzentrationen der Prüfsubstanzen wurden eingesetzt: Sphinganin 0,3 µg/ml; Kreatin 10 µg/ml; Koffein 10 µg/ml. Alle Kultivierungen wurden in Gegenwart von 0,1% (v/v) Ethanol durchgeführt und eine entsprechende Vehikel-Kontrolle mitgeführt. Nach 24-stündiger Inkubation wurde ein MTT-Assay durchgeführt. Die Aktivität im MTT-Test zeigt die Aktivität der mitochondrialen Dehydrogenase an und ist ein Marker für die Zellvitalität bzw. die Proliferationskapazität. Zunächst wurde eine 10 mg/ml Stammlösung des MTT-Reagenz (Thiazolyl Blue Tetrazolium Bromide, Sigma Aldrich M5655) in PBS angesetzt. Die Stammlösung wurde anschliessend mit Wachstumsmedium auf eine Endkonzentration von 1 mg/ml MTT-Reagenz verdünnt. Die Zellen wurden mit PBS (ohne Mg²⁺ und Ca²⁺) gewaschen und mit 1 ml Wachstumsmedium mit MTT-Reagenz für 3 h bei 37 °C inkubiert. Dann wurde das Medium vollständig entfernt und zur Extraktion des gebildeten blauen Formazan-Farbstoffs je 1 ml Isopropanol pro Well zugegeben. Anschliessend wurde für eine h bei Raumtemperatur auf einem Vertikalschüttler inkubiert. Hernach wurden aus jedem Well 200 µl des Isopropanolextrakts in ein Well einer 96-Well-Mikrotiterplatte überführt. Die Absorption der Extrakte wurde bei einer Wellenlänge von 550 nm spektrophotometrisch bestimmt. Die optische Dichte der Extrakte von den nur mit dem Vehikel behandelten Zellen wird als Referenz (100%) angesehen, alle anderen Messwerte werden relativ zur Referenzprobe quantifiziert. Die Resultate sind in Abbildung 1 gezeigt. Sphinganin (103,9%), Kreatin (104,0%) und Koffein (102,5%) zeigen alleine eine moderate Stimulation der Zellproliferation im Vergleich zur Vehikel-Kontrolle. Erstaunlicherweise wird durch die Kombination von Sphinganin mit Kreatin (135,5%) bzw. mit Koffein (122,1%) jeweils ein deutlich stärkerer positiver Effekt auf die Zellproliferation erzielt.

### Beispiel 2: Verbesserte sensorische Eigenschaften kosmetischer Formulierungen enthaltend Sphinganin in Kombination mit weiteren kosmetischen Wirkstoffen.

Kosmetische Cremeformulierungen enthaltend mindestens einen kosmetischen Wirkstoff ausgewählt aus der Gruppe von Sphinganin, Kreatin, Kreatinin, Koffein, Carnitin, Biotin, Arjunolsäure, Xymenynic-Säure, Minoxidil und Arginin wurden in einem Panel-Test eingesetzt, um die sensorischen Eigenschaften der applizierten Cremeformulierungen zu evaluieren. Die Zusammensetzung der Cremeformulierungen ist in Tabelle 1 gezeigt.
Zur Herstellung der Formulierungen wurden dem Fachmann bekannte, übliche Formulierungsverfahren eingesetzt.

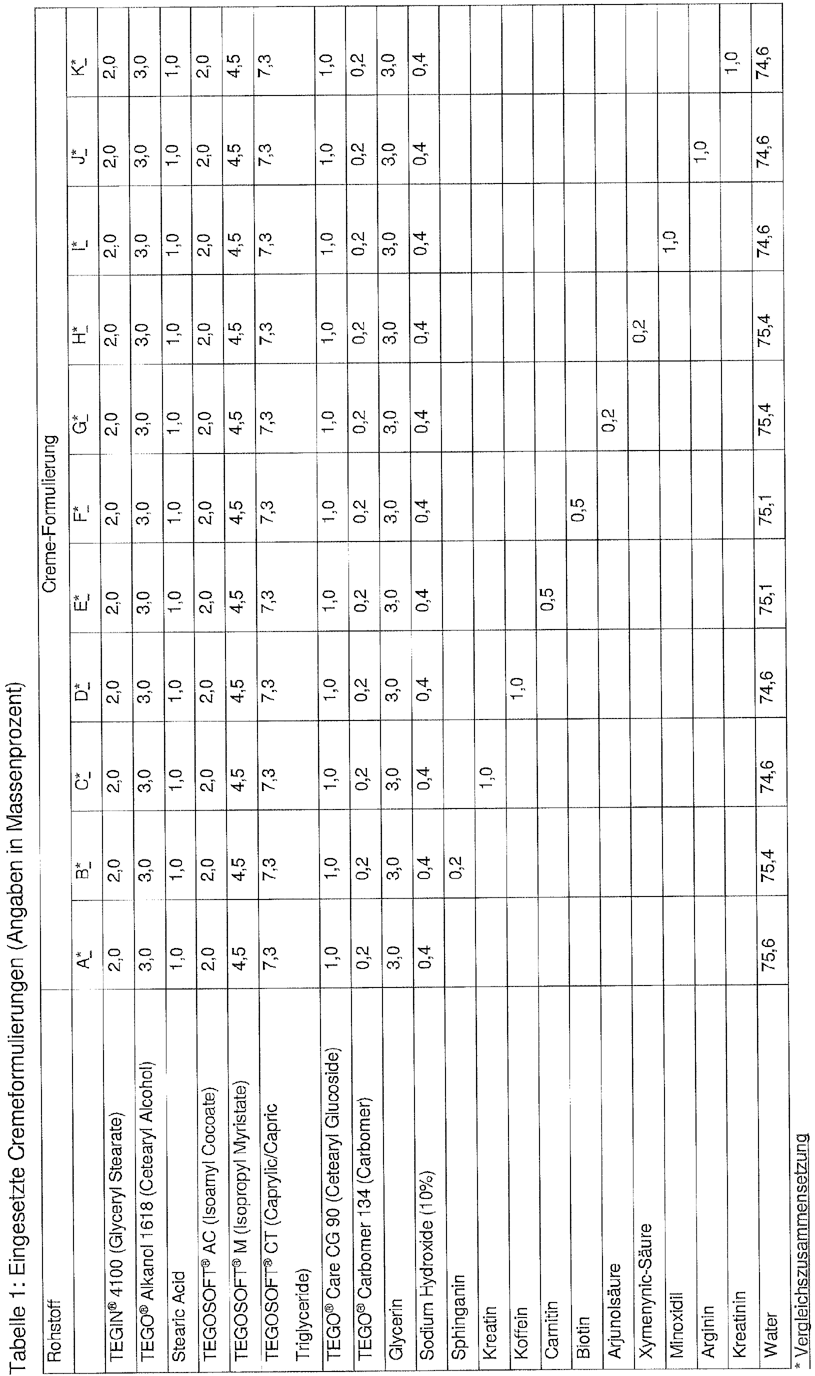

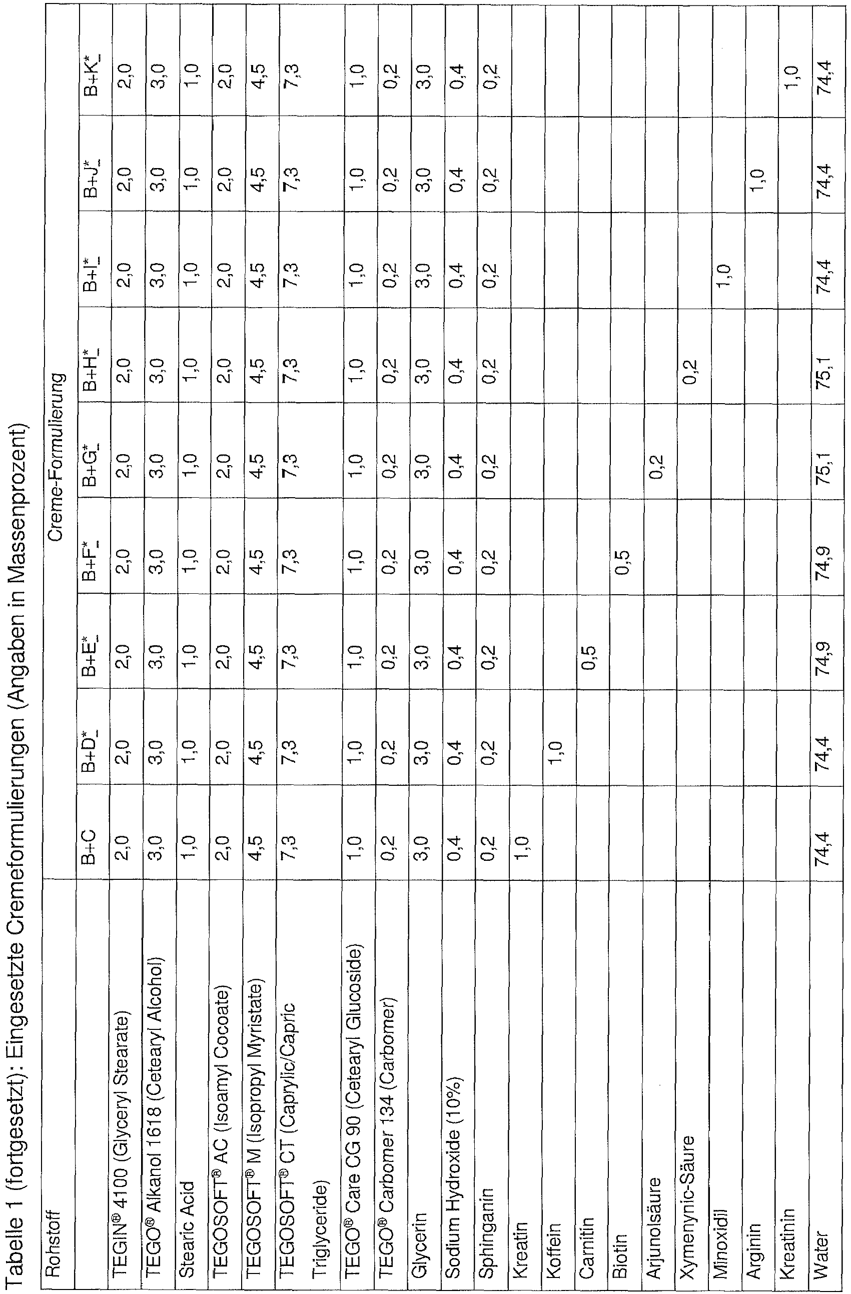

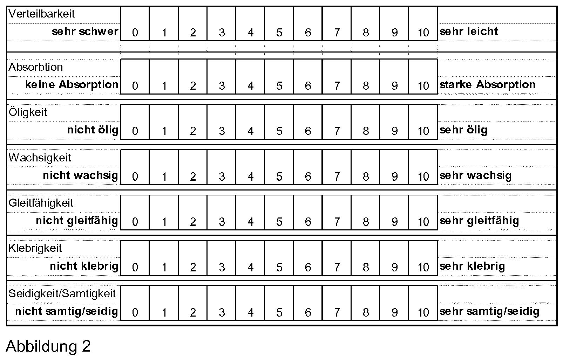

Jede Formulierung wurde von demselben Panel, bestehend aus 20 trainierten Experten, begutachtet. Konkret wurden jeweils 30 mg der Cremes auf zuvor mit Ethanol gesäuberte und mittels Stempel markierte Areale (Fläche 7 cm²) der Innenseite der Unterarme appliziert. Die Applikation erfolgte nach einem standardisierten Muster, d.h. jeder Panel-Teilnehmer verteilte zunächst das jeweilige Creme-Muster auf dem Testfeld durch kreisartige Bewegungen mit dem Zeigefinger bis zur Absorption des Testmusters. Nach den ersten 5 Kreisen wurde die Verteilbarkeit evaluiert, nach Beendigung der Verteilung wurden Absorption, Öligkeit, Wachsigkeit, Gleitfähigkeit, Klebrigkeit und Seidigkeit/Samtigkeit evaluiert. Anschliessend vergaben die Panel-Teilnehmer Zahlenwerte, welche die sensorischen Eigenschaften der jeweiligen Cremeformulierung beschreiben. Die Bedeutung der zugewiesenen Punkte wird in Abbildung 2 erläutert. In Tabelle 2 sind die durchschnittlich vergebenen Punkte (n = 20) für die Cremes mit den benannten Wirkstoffen gezeigt.

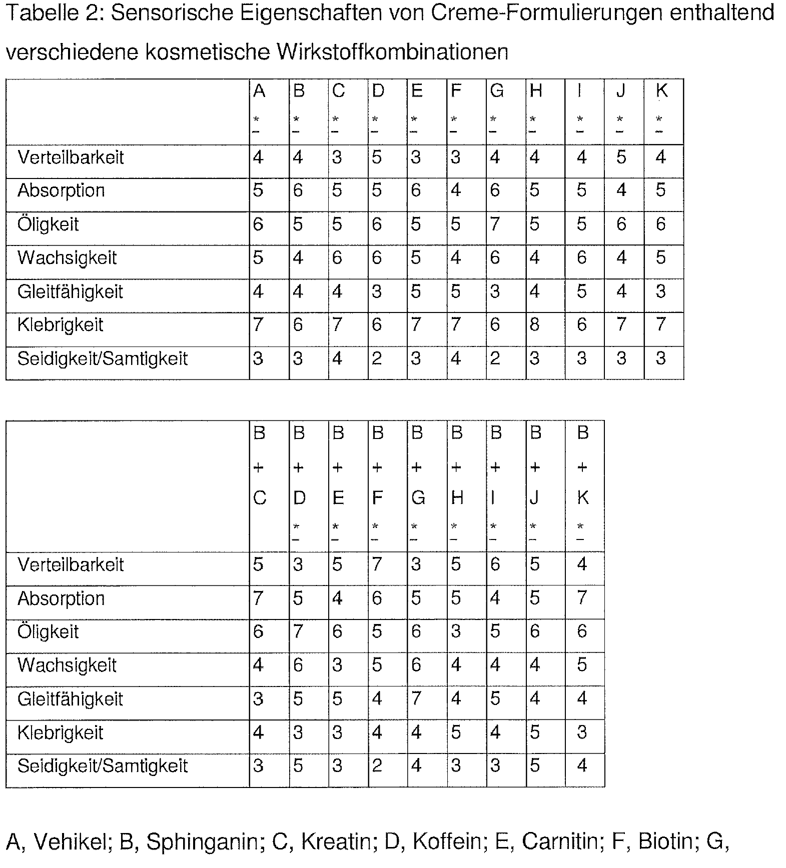

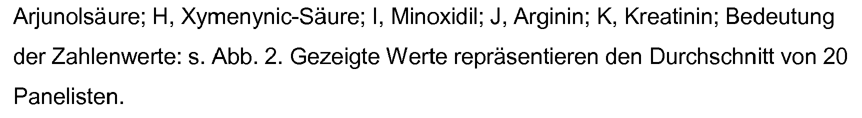

Überraschenderweise führte die paarweise Kombination von Sphinganin mit den in Tabelle 1 aufgeführten kosmetischen Wirkstoffen zu einem deutlich verbesserten sensorischen Profil bzw. Hautgefühl. Am auffälligsten war dies im Falle der Klebrigkeit. Die Creme mit Sphinganin als einzigem Wirkstoff zeigte hier nur eine leichte Verbesserung des Klebrigkeits-Parameters (6 Punkte i. Vgl. zur Vehikelkontrolle mit 7 Punkten). In der paarweisen Kombination mit den benannten Wirkstoffen hingegen wurden für die Klebrigkeit im Durchschnitt Werte zwischen 3 und 5 erzielt, d.h. die Klebrigkeit wurde i. Vgl. zum Vehikel jeweils deutlich herabgesetzt. Positive Effekte auf das Hautgefühl zeigten sich zudem auch bei folgenden Wirkstoffkombinationen (jeweils im Vergleich. zum Vehikel):
- Sphinganin und Biotin sowie Sphinganin und Minoxidil verbesserten die Verteilbarkeit der Creme auf der Haut (7 bzw. 6 gegenüber 4 Punkten)
- Sphinganin und Kreatin sowie Sphinganin und Kreatinin verstärkten die Absorption, und damit das Einziehen der Creme in die Haut (jeweils 7 gegenüber 5 Punkten)
- Sphinganin und Xymenynic-Säure verminderten die Öligkeit der Creme auf der Haut (3 gegenüber 6 Punkten)
- Sphinganin und Carnitin verminderten die Wachsigkeit der Creme auf der Haut (3 gegenüber 5 Punkten)
- Sphinganin und Arjunolsäure verbesserten die Gleitfähigkeit der Creme auf der Haut (7 gegenüber 4 Punkten)
- Sphinganin und Koffein sowie Sphinganin und Arginin verbesserten die Seidigkeit der Creme auf der Haut (jeweils 5 gegenüber 3 Punkten)

Die kosmetischen Formulierungen enthaltend Sphinganin in Kombinationen mit jeweils einem der oben aufgeführten Wirkstoffe zeigten somit in Anwendungstests überraschenderweise ein verbessertes sensorisches Profil gegenüber Formulierungen, die nur Sphinganin oder einen der einzelnen anderen Wirkstoffe enthalten.

## Patentansprüche

1. Zusammensetzung enthaltend
A) Sphinganin und
B) mindestens einen Wirkstoff ausgewählt aus der Gruppe Kreatin, Kreatin-Hydrate und Di-Natrium Phospho-Kreatin
zur Verwendung zur Stimulation des Haar-Wachstums.

2. Zusammensetzung enthaltend
A) Sphinganin und
B) mindestens einen Wirkstoff ausgewählt aus der Gruppe Kreatin, Kreatin-Hydrate und Di-Natrium Phospho-Kreatin
zur Verwendung zur Behandlung von Haarausfall.

3. Zusammensetzung zur Verwendung zur Stimulation des Haar-Wachstums gemäß Anspruch 1 oder zur Verwendung zur Behandlung von Haarausfall gemäß Anspruch 2, **dadurch gekennzeichnet, dass** Komponente A) in einer Konzentration von
0,01 Gew.-% bis 5,0 Gew.-%,
enthalten ist.

4. Zusammensetzung zur Verwendung zur Stimulation des Haar-Wachstums gemäß mindestens einem der vorherigen Ansprüche oder zur Verwendung zur Behandlung von Haarausfall gemäß Anspruch 2 oder 3, **dadurch gekennzeichnet, dass** Komponente B) in einer Konzentration von
0,01 Gew.-% bis 20,0 Gew.-%,
enthalten ist.

5. Zusammensetzung zur Verwendung zur Stimulation des Haar-Wachstums gemäß mindestens einem der vorherigen Ansprüche oder zur Verwendung zur Behandlung von Haarausfall gemäß mindestens einem der Ansprüche 2 bis 4, **dadurch gekennzeichnet, dass** das Gewichtsverhältnis der Komponente A) zu der Komponente B) in der Zusammensetzung
1 zu 1 bis 1 zu 2000
beträgt.

6. Nicht-therapeutische kosmetische Verwendung einer Zusammensetzung enthaltend
A) Sphinganin und
B) mindestens einen Wirkstoff ausgewählt aus der Gruppe Kreatin, Kreatin-Hydrate und Di-Natrium Phospho-Kreatin
zur Stimulation des Haar-Wachstums.

## Claims

1. Composition comprising
A) sphinganine and
B) at least one active ingredient selected from the group of creatine, creatine hydrate and disodium phosphocreatine
for use for the stimulation of hair growth.

2. Composition comprising
A) sphinganine and
B) at least one active ingredient selected from the group of creatine, creatine hydrate and disodium phosphocreatine
for use for the treatment of hair loss.

3. Composition for use for the stimulation of hair growth according to Claim 1 or for use for the treatment of hair loss according to Claim 2, **characterized in that** component A) is present at a concentration of
0.01% by weight to 5.0% by weight.

4. Composition for use for the stimulation of hair growth according to at least one of the preceding claims or for use for the treatment of hair loss according to Claim 2 or 3, **characterized in that** component B) is present at a concentration of 0.01% by weight to 20.0% by weight.

5. Composition for use for the stimulation of hair growth according to at least one of the preceding claims or for use for the treatment of hair loss according to at least one of Claims 2 to 4, **characterized in that** the ratio by weight of component A) to component B) in the composition is from 1:1 to 1:2000.

6. Non-therapeutic cosmetic use of a composition comprising
A) sphinganine and
B) at least one active ingredient selected from the group of creatine, creatine hydrate and disodium phosphocreatine
for the stimulation of hair growth.

## Revendications

1. Composition contenant
A) de la sphinganine et
B) au moins un principe actif choisi dans le groupe suivant
créatine, hydrates de créatine et phosphocréatine disodique
pour une utilisation pour la stimulation de la croissance des cheveux.

2. Composition contenant
A) de la sphinganine et
B) au moins un principe actif choisi dans le groupe suivant
créatine, hydrates de créatine et phosphocréatine disodique
pour une utilisation pour le traitement de la chute des cheveux.

3. Composition pour une utilisation pour la stimulation de la croissance des cheveux selon la revendication 1 ou pour une utilisation pour le traitement de la chute des cheveux selon la revendication 2, **caractérisée en ce que** le composant A) est contenu en une concentration de 0,01 % en poids à 5,0 % en poids.

4. Composition pour une utilisation pour la stimulation de la croissance des cheveux selon au moins une des revendications précédentes ou pour une utilisation pour le traitement de la chute des cheveux selon la revendication 2 ou 3, **caractérisée en ce que** le composant B) est contenu en une concentration de
0,01 % en poids à 20,0 % en poids.

5. Composition pour une utilisation pour la stimulation de la croissance des cheveux selon au moins une des revendications précédentes ou pour une utilisation pour le traitement de la chute des cheveux selon au moins une des revendications 2 à 4, **caractérisée en ce que** le rapport pondéral du composant A) sur le composant B) dans la composition est de 1 sur 1 à 1 sur 2000.

6. Utilisation cosmétique non-thérapeutique d'une composition contenant
A) de la sphinganine et
B) au moins un principe actif choisi dans le groupe suivant
créatine, hydrates de créatine et phosphocréatine disodique
pour la stimulation de la croissance des cheveux.
